# EUROPEAN PATENT APPLICATION

(11) **EP 1 213 360 A1**
(43) Date of publication of application: **12.06.2002**
(21) Application number: 01305312.9
(22) Date of filing: 19.06.2001
(51) Int. Cl.: C12Q 1/68, G06F 19/00

(54) **Method of identifying cells using DNA methylation patterns**

(30) Priority: 07.12.2000 JP 2000372954
(71) Applicant: The University of Tokyo, Tokyo 113-8654 (JP)
(72) Inventor: Shiota, Kunio, Inba-gun, Chiba, 270-1423 (JP); Tanaka, Satoshi, Wako-shi, Saitama 351-0114 (JP); Ohgane, Jun, Tokyo, 173-0016 (JP); Hattori, Naka, Tokyo 160-0003 (JP)
(74) Representative: Kremer, Simon Mark

(57) **Abstract**

The present invention provides a method of identifying a cell, tissue or nucleus, comprising collecting information on the methylation pattern of DNA isolated from the cell, tissue or nucleus and analyzing the resultant information.

## Description

### BACKGROUND OF THE INVENTION

### 1. Field of the Invention

The present invention relates to a method of identifying cells, tissues or nuclei using DNA methylation patterns.

### 2. Description of the Prior Art

Conventionally, types of cells have been identified using morphological characteristics and several molecules produced in cells (such as specific proteins or sugar chains) as indicators. For example, those cells that have an elongated shape like an axon and those cells that are expressing nerve fiber proteins can be judged nerve cells. Thus, in analyzing tissue or cell samples from normal individuals, traditional procedures of examining morphologies or several molecules have been used.

However, traditional procedures based on the morphology with checking few marker molecules will not be enough in the production of nerve cells or other cells for transplantation by, for example, inducing from embryonic stem cells in culture, there is a possibility that the produced cells might not exhibit expected functions when transplanted or a possibility that the growth of the produced cells might become uncontrollable after transplantation. It should also be taken into consideration that morphologies of cells change easily under various culture conditions. Thus, establishment of a cell identification method more accurate than the conventional, traditional method has been desired.

### OBJECTS AND SUMMARY OF THE INVENTION

It is an object of the invention to provide a method of identifying cell, tissues or nuclei using DNA methylation patterns.

As a result of intensive and extensive researches toward the solution of the problem, the present inventor has focused attention at the fact that DNA methylation patterns differ depending on types of cells, and found that it is possible to identify a cell, tissue or nucleus by analyzing information on the DNA methylation pattern of the cell, tissue or nucleus. Thus, the present invention has been achieved.

The present invention relates to a method of identifying a cell, tissue or nucleus, comprising collecting information on the methylation pattern of DNA isolated from the cell, tissue or nucleus and analyzing the resultant information.

Further, the present invention relates to a method of using information on the methylation pattern of DNA isolated from a test cell, tissue or nucleus as an indicator for producing a cell, tissue or nucleus of interest.

Further, the present invention relates to a method of specifying gene regions indispensable for producing a cell, tissue or nucleus of interest, using information on the methylation pattern of DNA isolated from a test cell, tissue or nucleus as an indicator.

Further, the present invention relates to a computer-readable record medium in which a program that permits a computer to function as an identification system for cells, tissues or nuclei has been recorded. The identification system comprises:
(a) means for analyzing information on the methylation pattern of DNA isolated from a test cell, tissue or nucleus; and
(b) means for identifying the cell, tissue or nucleus using the analysis results as an indicator.

The present specification encompasses the contents of the specification and drawings of Japanese Patent Application No. 2000-372954 based on which the present application claims priority.

### BRIEF DESCRIPTION OF THE DRAWINGS

Figure 1. Schematic diagram of cell type-specific methylation pattern at gene loci. Genomic DNA of three independent cell types by lines. Numbers 1-8 indicate genes in the genome. Ms in the circles show methylated loci in the genes 1-8, and each cell type has its own methylation patterns at genes 1-8.
Figure 2. Schematic diagram of the whole RLGS procedure. In this diagram, enzyme A is *Not* I or *Bss* HII, enzyme B is *Pvu* II or *Eco* RV and enzyme C is *Pst* I, *Hin*f I or *Mbo* I. Solid circles at the enzyme A site indicate ³²P incorporated. The length of the 1st D reflects the distance from enzyme A to B, and that of the 2nd D reflects the distance from enzyme A to C.
Figure 3. Whole RLGS profile for the kidney of C57BL6 mouse using an enzyme combination of *Not* I, *Pvu* II and *Pst* I. In the autoradiogram, 167 spots showing tissue-and cell type-specific appearances are indicated by circles.
Figure 4. Representative spots showing tissue- and cell type-specific appearances. Of the 167 tissue- and cell type-specific spots, 15 spots are indicated by arrows in the enlarged view of samples subjected to RLGS analysis.
Figure 5. Summary of 167 specific spots analyzed. Closed circles (●) indicate clear appearance of corresponding spot. Double circles (ⓞ) indicate weak appearance of spots. Open circles (○) indicate disappearance of spots. Bars (-) indicate spots that were impossible to discriminate.
   EU, Embryonic stem cell (Undifferentiated); ED, Embryonic stem cell (Differentiated); TU, Trophoblast stem cell (Undifferentiated); TD, Trophoblast stem cell (Differentiated); PL, Placenta; KD, Kidney; SP, Sperm; BR, Brain.
Figure 6. Block diagram of the system identifying tissue or cell type based on the spot pattern specific to them.
Figure 7. Flow chart showing an example of cell type identification processing according to the identification program.

### DETAILED DESCRIPTION OF THE INVENTION

In mammals including human, cells once differentiated during the course of development inherit the same characters as those of their parent cells and, usually, such characters are retained throughout the lives of individuals. Genomic DNA in each cell posseses basically the same set of genetic information regardless of the types of cells in an individual, but the set of genes expressed is restricted by the types of cells. The present inventor has found that there exist unique genomic DNA methylation patterns depending on the types of cells. Since the methylation of genomic DNA is related to the gene activity including gene-silencing, DNA methylation patterns specific to cells or tissues are considered to function as a mechanism for memorizing gene expressions inherent in individual types of cells.

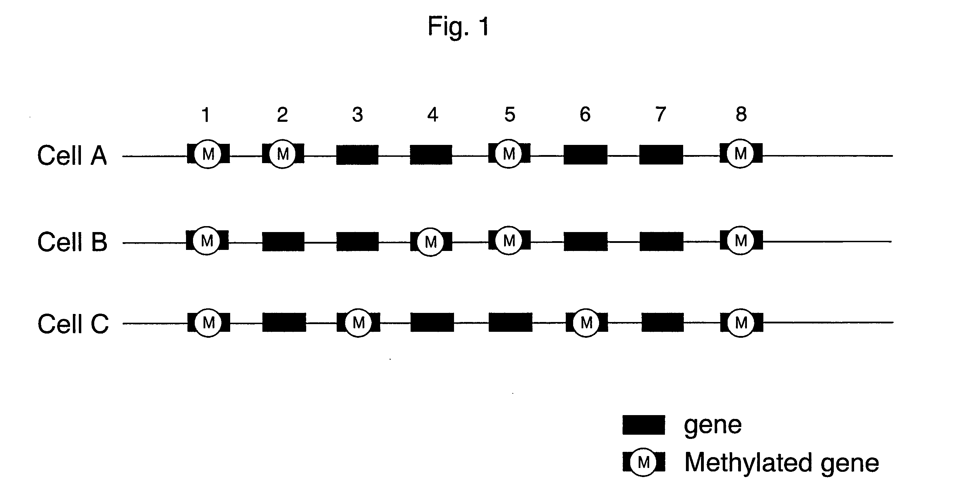

The method of the invention utilizes the fact that methylation patterns appearing on genomic DNA differ depending on types of cells, tissues or nuclei (hereinafter, sometimes referred to as "cells, etc."). The method of the invention is characterized by analyzing information on the methylation pattern of genomic DNA. The term "analyzing" used herein means one or both of the following (1) and (2): (1) to identify of which parts of DNA the methylation and/or non-methylation is specific to a test cell, tissue or nucleus; and (2) to detect the presence or absence of methylation in the specific parts of DNA to thereby identify the type of the cell, tissue or nucleus. For example, suppose there are three types of cells, A, B and C, and each of them has 8 genes within a specific region of genomic DNA as illustrated in Fig. 1. Suppose that the results of examination of methylation in this region revealed that genes 1, 2, 5 and 8 are methylated in cell A; that genes 1, 4, 5 and 8 are methylated in cell B; and that genes 1, 3, 6 and 8 are methylated in cell C. The expression "gene X is methylated" (X represents the name of the gene or any number or symbol) used herein means that the carbon at the 5 position of cytosine is methylated in 5'-CG-3' sequences (hereinafter referred to as "CpG sequences") present in a specific region(s) of gene X. When these methylation patterns are compared, the methylation patterns (i.e., the presence or absence of methylation in CpG sequences) of gene 1 are common in all of the three cells, and so are the methylation patterns of genes 7 and 8. These genes cannot be discriminated with methylation patterns. However, within these three cells, the methylation of gene 2 is specific to cell A; the methylation of genes 3 and 6 are specific to cell C; and the methylation of gene 4 is specific to cell B. Thus, within these three cells, a cell having information that gene 2 is methylated can be identified as cell A. Further, more accurate identification can be made by combining information on methylation/non-methylation patterns of genes 1 through 8. Thus, by utilizing the fact that DNA methylation differs depending on types of cells and analyzing information on DNA methylation, it is possible to identify a given cell.

The term "information on methylation pattern" used herein means information on which specific loci in the genomic DNA of a cell are methylated. This information can be obtained by detecting methylation of the DNA. The identification symbols or numbers of sequences on the DNA (e.g., the gene numbers 1 through 8 in Fig. 1) may be assigned in the order in which genes are located on the genome, or may be assigned regardless of that order on the genome as long as there is some specific rule in the assignment (i.e., as long as corresponding genes in different cells have the same symbol or number).

A preferred example of DNA that the method of the invention can handle may be genomic DNA obtained from cells, tissues or nuclei derived from animals. Tissues include various organs. Specifically, neural tissues such as brain, spinal cord; digestive organs such as esophagus, stomach, small intestine, large intestine; respiratory organs such as lung, bronchus; reproductive organs such as testis, ovary, uterus, placenta; urinary organs such as kidney, urinary bladder; and hematopoietic organs such as bone marrow, blood may be enumerated. Specific example of cells includes embryonic stem cell, trophoblast stem cell, bone marrow stem cell and nerve stem cell. These cells may be cells obtained from the above-mentioned tissues through protease treatment, or they may be cultured cells. Nuclei may be obtained by centrifuging extracts from the above-mentioned cells to thereby separate nuclear fractions from other fractions.

Examples of genomic DNA from animals include genomic DNA from human, monkey, dog, mouse, rat or cow.

Genomic DNA may be prepared by any of the conventional methods (Okazaki, Y. et al., Proc. Natl. Acad. Sci. USA (1995)). For example, a tissue sample is ground down into powder and then suspended in an appropriate cell lysis solution (containing protease). The resultant lysate is subjected to extraction with, e.g., phenol/chloroform/isoamyl alcohol, followed by precipitation of genomic DNA in ethanol.

Methods for detecting methylation in the obtained DNA are not particularly limited. Any method, e.g., the RLGS technique, MS-PCR technique, Southern blotting, or CpG island microarray technique, may be used to identify methylation patterns. It should be noted that techniques useful in the present invention for obtaining information on methylation are not limited to the above-mentioned techniques. Any technique may be used as long as information on methylation can be obtained with it. Hereinbelow, the method of the invention will be described with reference to the RLGS technique, the MS-PCR technique, Southern blotting, and CpG island microarray technique as non-limiting examples.

### (1) RLGS Technique

The RLGS (restriction landmark genomic scanning) technique is a widely known technique in which recognition sites of restriction enzymes are used as landmarks for detecting methylation. Briefly, DNA is extracted from a test cell or tissue and then digested with a methylation-sensitive restriction enzyme to thereby produce DNA fragments. These fragments are labeled at the 5' end with a labeling material (e.g., ³²P) and separated by the first-dimensional electrophoresis. The resultant DNA fragments are digested with restriction enzymes other than the above methylation-sensitive restriction enzyme and subjected to the second-dimensional electrophoresis. Then, spots are analyzed by autoradiography, etc. Subsequently, a database of spot patterns specific to the test cell or tissue is prepared. By producing such spot patterns for a cell of interest and for cells to be used for comparison and then comparing those spot patterns, the cell of interest is identified. In the present invention, the state of methylation in several thousand regions of genomic DNA can be analyzed at one time by using a methylation-sensitive restriction enzyme.

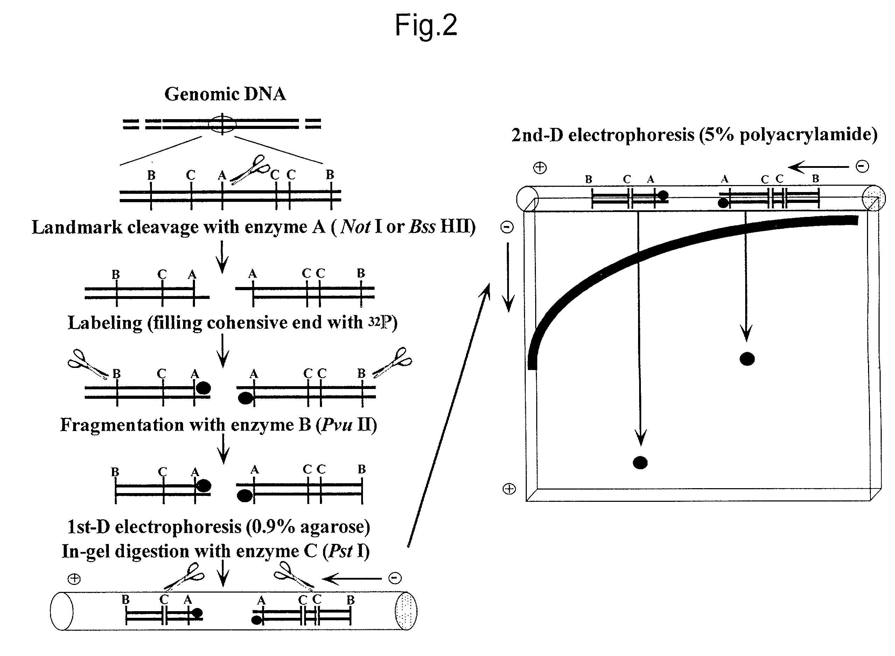

More specifically, the extracted genomic DNA is digested with a methylation-sensitive restriction enzyme, a first restriction enzyme [see Fig. 2a and Fig. 2b]. The methylation-sensitive restriction enzyme is an enzyme that cuts the DNA at site "A" in Fig. 2a (hereinafter referred to as "restriction enzyme A"). When the 5 position of cytosine is methylated (modified) in CG dinucleotide, this enzyme becomes unable to cut the site due to the effect of the methylation.

As restriction enzyme A, such an enzyme is preferable that generates DNA fragments of more than 100 kb in average as a result of digestion (i.e., recognizes a restriction site that is present in intervals of more than 100 kb in average) and recognizes 6 to 8 bases. Specific examples of restriction enzyme A include *Not*I, *Bss*HII and *Sal*I.

Subsequently, the site cut by the above restriction enzyme A is labeled by introducing a labeled nucleotide [see Fig. 2c]. Examples of labeling materials useful in the invention include radioisotopes such as [α-³²P] dCTP, [α-³²P] dGTP, and fluorescent dyes such as tetramethyl-rhodamine-6-dUTP, fluorescein-12-dUTP. The introduction of a labeled nucleotide may be performed using a commercial kit (e.g., Sequenase ver. 2: New England Biolab).

In order to cut the restriction fragments produced by restriction enzyme A into still shorter fragments, the fragments are digested with a second restriction enzyme different from restriction enzyme A [see Fig. 2d]. The second restriction enzyme is an enzyme that generates DNA fragments of several to several ten kb in average as a result of digestion (i.e., recognizes a restriction site that is present in intervals of several to several ten kb in average) and recognizes 4 to 6 bases. [This enzyme cuts DNA fragments at site "B" in Figure 2 and is referred to as "restriction enzyme B".] Specific examples of restriction enzyme B include *Pvu*II and *Eco*RV. After digestion with restriction enzyme B, the resultant fragments are subjected to the first-dimensional fractionation [see Fig. 2e].

After completion of the fractionation, the tube used is dipped in a solution of a third restriction enzyme that is different from either restriction enzyme A or B used above, to thereby digest the product from the first-dimensional fractionation. The third restriction enzyme has a higher restriction frequency than restriction enzymes A and B, and recognizes a restriction site that is present in intervals of several hundred bp in average. [This enzyme cuts DNA fragments at site "C" in Figure 2 and is referred to as "restriction enzyme C".] Restriction enzyme C may be an enzyme that recognizes 4 to 6 based. Specific examples of restriction enzyme C include *Pst*I, *Hin*fI and *Mbo*I.

As a result of digestion with restriction enzyme C, fragments sandwiched with restriction sites A and B (hereinafter referred to as "A-B fragments") are cut into fragments sandwiched with restriction sites A and C (hereinafter referred to as "A-C fragments") and fragments sandwiched with restriction sites C and B (hereinafter referred to as "B-C fragments"). The average length of each of these two types of fragments becomes several hundred bp or less. Then, these fragments are subjected to the second-dimensional fractionation [see Fig. 2f]. As a method of the second-dimensional fractionation, 5% polyacrylamide gel electrophoresis may be used, for example.

The detection of spots is performed by a technique suitable for the labeling material used. For example, when ³²P is used as a labeling material, detection may be carried out by autoradiography. When a fluorescent dye is used as a labeling material, detection may be carried out with a fluorescence image analyzer (e.g., Molecular Imager FX: BioRad).

The positions of the obtained spots are expressed with the distance in X direction (i.e., the direction of the the first-dimensional electrophoresis) from the origin and the distance in Y direction (i.e., the direction of the second-dimensional electrophoresis) from the origin, e.g., (X₁, Y₁), (X₂, Y₂), ..... (Xₙ, Yₙ). The abscissa X reflects the distance from restriction site A to restriction site B (the length of A-B fragment), and the ordinate Y reflects the distance from restriction site A to restriction site C (the length of A-C fragment). Thus, using these coordinates, identification marks can be given to specific gene regions in genomic DNA (these gene regions are not necessarily in the order in which they are aligned in the gnomic DNA), and then patterns of those spots can be analyzed. As a result, the test cell can be identified. For the purpose of simplification, identification marks may be serial numbers or symbols in addition to coordinates.

The analysis of the methylation pattern thus obtained by the RLGS technique may be performed as described below, for example.

There are two kinds of spots: spots that appear in every cell or tissue tested and spots that appear (or do not appear) depending on the type of the cell or tissue tested. For example, when detection of spots at specific positions has been performed in 8 cell types, there are (i) positions at which a spot always appears in all of the 8 cell types and (ii) positions at which a spot appears in 1 to 7 cell types. In the present invention, the spots constantly appearing (the spots at positions (i)) are excluded from analysis. Those spots whose appearance varied depending on the types of cells or tissues tested (the spots at positions (ii)) are selected for analysis. Subsequently, identification marks (serial numbers or indications with coordinates) are given to those positions at which spot appearance varied depending on the types of cells or tissues.

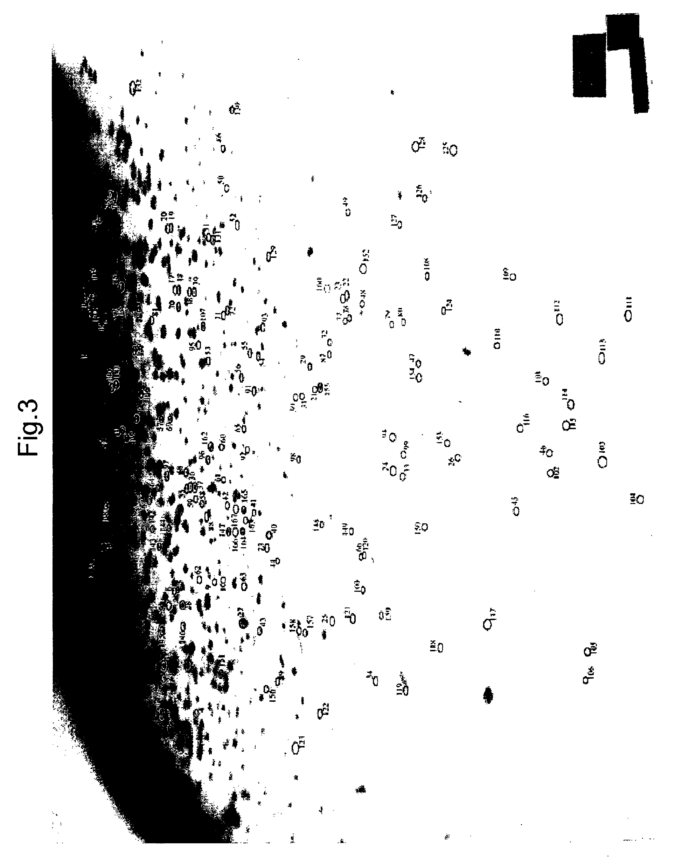

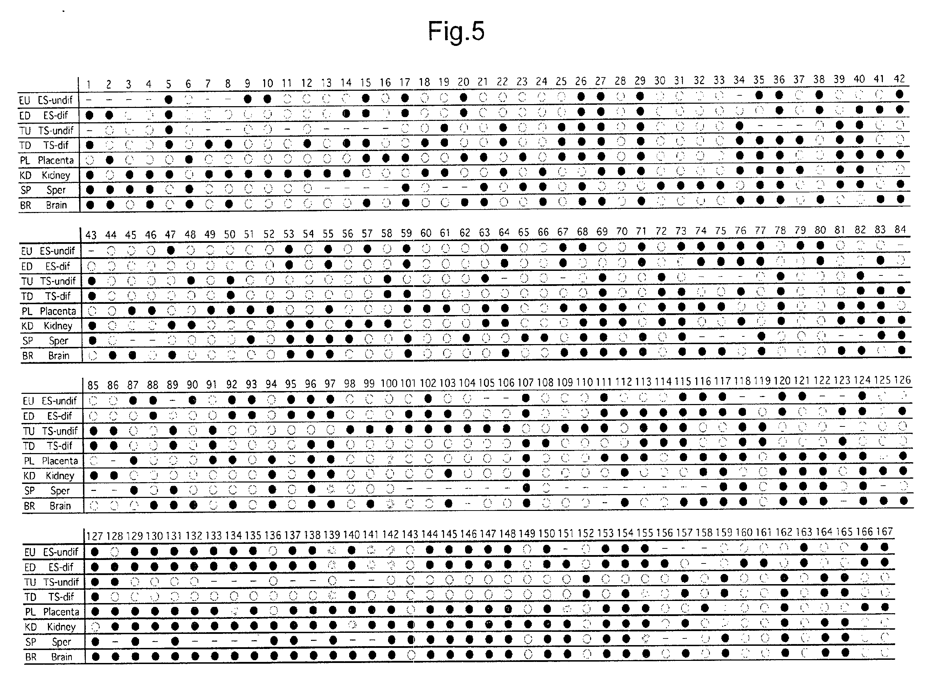

An embodiment of the present invention in which methylation patterns of 8 cell types, etc. are produced as shown in Example 1 will be described below. For each of the 8 cell types, etc., spot patterns at about 1,000 positions are generated. Among these positions, 167 positions have different spot appearance patterns depending on the cells, etc. Thus, these positions are selected as targets of analysis, and the remaining positions are excluded from the analysis since spots appear constantly in all of the 8 cell types, etc. However, they serve as markers for identifying the positions of those spots detected specifically. The spots shown in Fig. 3 represent the methylation pattern of the DNA derived from C57BL/6 mouse kidney. The above-described 167 positions are indicated with mark "○" on the pattern. Such a pattern was produced for each of the remaining 7 cell types, etc. and the 167 positions were marked (Fig. 5).

Whether or not a spot is specific to the test tissue or cell is judged as follows. Briefly, the presence or absence of a spot is detected at positions (to which identification numbers have been given) on patterns produced in advance for any tissues or cells, and at corresponding positions on a pattern produced for the test tissue or cell. The results are compared to thereby judge whether the spot is specific to the test tissue or cell. When the presence or absence of a spot at each position having an identification number is shown schematically as illustrated in Fig. 5, it is possible to judge whether a certain spot is specific to the tissue or cell tested. For example, at position 79, a spot is appearing only in embryonic stem cell (undifferentiated) among the 8 cells, etc. tested. Thus, a cell that has obtained a spot at position 79 can be identified as embryonic stem cell (undifferentiated). In other words, it can be said that a spot appears at position 79 if the test cell is embryonic stem cell (undifferentiated).

However, it is not necessary to select only one spot specific to the particular cell or tissue as a spot to be analyzed. It is possible to identify cells or tissues with a combination of a plurality of spots. For example, when attention is focused on spots at positions 79 and 80 in Figure 5, a cell that has a spot at both positions 79 and 80 can be identified as undifferentiated embryonic stem cell; and a cell that does not have a spot at position 79 but has one at position 80 can be identifies as differentiated embryonic stem cell. Similar analysis can be made with a combination of three or more spots. The number of samples is not limited to the 8 cell types, etc. as illustrated in the above example. With a greater number of samples, it is possible to identify cells, etc. more precisely and accurately. This means that there is a possibility that a spot once found specific to a particular cell within a certain number of cells may be revealed not specific to the cell when examined within a greater number of cells. Therefore, it is preferable to analyze whether a methylation pattern is specific or not using as many samples as possible. Alternatively, it is preferable to limit or select test samples depending on the purpose of identification and to make such analysis within that range.

If a spot obtained is specific to a certain tissue or cell, the spot becomes a source of information derived from the tissue or cell (the position, intensity, etc. of the spot). Therefore, the presence or absence of such spots (e.g., the information contained in Fig. 5) is accumulated in a database. The construction of such a database is performed by digitalizing individual information so that a computer can analyze the information. For example, the digitalization of positions is performed using coordinates or identification numbers; and the digitalization of the intensity of spots is performed by quantitatively determining spot intensities.

By accumulating such information, it is possible to specify the type of a cell by comparing the spot pattern obtained from the cell with spot pattern information accumulated in the database, even if the type or origin of the cell is unknown. In order to compare the relevant spot pattern with the spot pattern information in the database, a computer program for identifying cells, etc. may be used. Further, by classifying cells, etc. into several categories in the database, a systematic identification can be made.

### (2) MS-PCR Technique

In the PCR technique, specific primers are designed and synthesized so that a particular gene in genomic DNA is amplified. Using these primers, a PCR is performed with the particular gene as a template. When the gene regions of the genomic DNA have been digested with a methylation-sensitive restriction enzyme before the amplification, methylated genes are not cut while unmethylated genes are cut. These genes are amplified by PCR, and the amplified fragments are separated by electrophoresis. Then, the resultant bands are examined. If the test gene is methylated, bands are observed. If the test gene is unmethylated, no bands are observed. Using this fact, whether the test gene is methylated or not can be ascertained.

For example, when the methylation of gene 1 of cell A shown in Fig. 1 is examined, first, a pair of specific primers are designed so that gene 1 is amplified by PCR. The specific primers (forward and reverse) may be selected from any regions of gene 1, or may be selected from a region adjacent to the 5' end and a region adjacent to the 3' end. The number of nucleotides for each primer is 10 to 35, preferably 20 to 30. These primers are designed so that the length of the resultant amplification fragments is 100-1000 bp, preferably 200-500 bp. Further, the primers are designed so that the resultant amplification fragments contain a recognition site of a methylation-sensitive restriction enzyme. If the nucleotide sequence of the gene to be amplified is unknown, the sequence can be determined with a commercial automated DNA sequencer (e.g., 373A DNA Sequencer: Perkin-Elmer).

A PCR is performed using any equipment (e.g., Robocycler: Stratagene) and under cycling conditions selected appropriately. After completion of the PCR, the amplified fragments are subjected to agarose gel electrophoresis, followed by examination of the resultant bands.

### (3) Southern Blotting

When genomic DNA is digested with a methylation-sensitive restriction enzyme, methylated restriction sites are not cut while unmethylated restriction sites are cut. The digested genomic DNA is separated by agarose electrophoresis. The DNA fragments are transferred onto a nylon membrane followed by hybridization with a ³²P-labeled gene-specific probe. Then, the presence or absence of methylation in the gene used as the probe can be detected using the difference in length of the detected bands.

### (4) CpG Island Array Technique

First, genomic DNA is digested with a restriction enzyme which does not contain methylatable sequences in its recognition site. Then, a linker containing a primer site for PCR is ligated to the digested genomic DNA. The linker-ligated DNA fragments are digested with a methylation-sensitive restriction enzyme and then amplified by PCR utilizing the primer site in the linker. At that time, unmethylated genes are cut between primers by the methylation-sensitive restriction enzyme, and not amplified by PCR. On the other hand, only methylated genes are amplified. Thus, if such a PCR reaction is performed using a combination of any two tissues or cells, the types of amplified genes are different because of the existence of methylated regions specific to respective tissues or cells. Only those genes that exhibit difference in methylation between the tissues or cells are selected by the subtraction method and used as probes. These probes are hybridized with a gene library to thereby confirm their nucleotide sequences. Thus, the genes can be identified.

### 2. Specification of Gene Regions and Use Thereof as an Indicator for Producing Cells, etc.

According to the present invention, it is possible to use information on DNA methylation patterns as an indicator for producing a cell, tissue or nucleus of interest. In other words, it is possible to specify those regions indispensable for producing a cell, tissue or nucleus of interest by utilizing the fact that methylation patterns differ depending on types of cells.

For example, suppose that analysis of methylation patterns revealed that cell A and cell B in Fig 1 are embryonic stem cells (ES cells) and that (i) when gene 2 is methylated and gene 4 is unmethylated, the cell is classified as differentiated stem cell (cell A) and (ii) when gene 2 is unmethylated and gene 4 is methylated, the cell is classified as undifferentiated stem cell (cell B). In this case, gene regions indispensable for producing an ES cell are gene 2 and gene 4. In the production of tissues or nuclei, indispensable gene regions may be specified in the same manner.

Methylation of genes is performed by treating DNA with a methyltransferase (e.g., *Sss*I or *Hpa*II methylase). In once differentiated cells, the methylation pattern thereof is transmitted to new cells through cell division in the following manner. Briefly, during the phase of DNA replication prior to cell division (i.e., S phase), methylated cytosine bases in the parent DNA strand are recognized by DNA methyltransferase, which then methylates the daughter DNA strand.

Thus, if the production of differentiated ES cells is intended, undifferentiated ES cells may be cultured after random methylation of genes thereof. The cultivation may be carried out in a medium, e.g., commonly used RPMI1640, DMEM, MEM or such a medium supplemented with bovine serum albumin, etc., according to conventional animal cell culture techniques. Subsequently, the resultant cells are subjected to random demethylation. Then, information on methylation patterns is analyzed as described above. Using the analysis results as an indicator, those cells in which gene 2 is methylated and gene 4 is demethylated are screened for and isolated. Thus, the cell of interest can be obtained.

In the present invention, the cell to be produced is not particularly limited. For example, embryonic stem cells, trophoblast stem cells, bone marrow stem cells and nerve stem cells may be enumerated as highly useful cells.

In the present invention, the tissue to be produced is not particularly limited. For example, neural tissues such as brain, spinal cord; digestive organs such as esophagus, stomach, small intestine, large intestine; respiratory organs such as lung, bronchus; reproductive organs such as testis, ovary, uterus, placenta; urinary organs such as kidney, urinary bladder; and hematopoietic organs such as bone marrow, blood may be enumerated. As a method for producing a tissue of interest, such a method may be employed in which cells are grown up to an appropriate number (10⁶ to 10⁷ cells, preferably 6x10⁶ to 10⁷ cells, per 25 ml of the medium) followed by conventional tissue culture for regeneration into a tissue.

Even when site-specific methylation or demethylation has become possible or when it has become possible to produce stem cells freely, the method of the present invention will still be able to contribute to the evaluation of safety of the produced stem cells when transplanted or to the improvement of efficiency of the cell production by determining whether the produced cells are stem cells or not or by evaluating the degree of the stem cells.

### 3. Cell Identification System

Hereinbelow, the identification system of the invention for cells, tissues or nuclei will be described. The identification system of the invention comprises:
(a) means for analyzing information on the methylation pattern of DNA isolated from a test cell, tissue or nucleus; and
(b) means for identifying the test cell, etc. using the analysis results as an indicator.

The analysis means described in (a) above is composed of means for detecting the methylation patterns of the genomic DNA isolated from the test cell, tissue or nucleus and other genomic DNAs isolated from other cells, tissues or nuclei, respectively, (also called "detection engine") and means for comparing the resultant values detected (also called "comparison engine").

The identification means described in (b) above is composed of means for judging the identity of the test cell, tissue or nucleus with one of the other cells, tissues or nuclei.

### (1) Detection Engine for DNA Methylation Patterns

In the present invention, the detection of DNA methylation patterns can be performed by digitalizing methylation patterns obtained as described above and using or applying the digitalized information.

### (2) Comparison Engine

Comparison engine accumulates (i) information on DNA methylation patterns of certain wild-type cells, tissues or nuclei; (ii) information on DNA methylation patterns of certain mutant cells, tissues or nuclei; or DNA methylation patterns of particular cells, tissues or nuclei; and (iii) information on the DNA methylation pattern of a cell, tissue or nucleus to be identified.

### (3) Cell Identification Engine

Cell identification engine is a means for judging the identity of the test cell, etc. with other cell, etc. based on the data obtained by the comparison engine (e.g., difference in the positions at which spots appear, or difference in spot or band intensities, etc.). In examining whether cell A is identical with cell B, for example, this engine judges to what extent the DNA methylation pattern of cell A may differ from that of cell B in order for cell A to be identified with cell B.

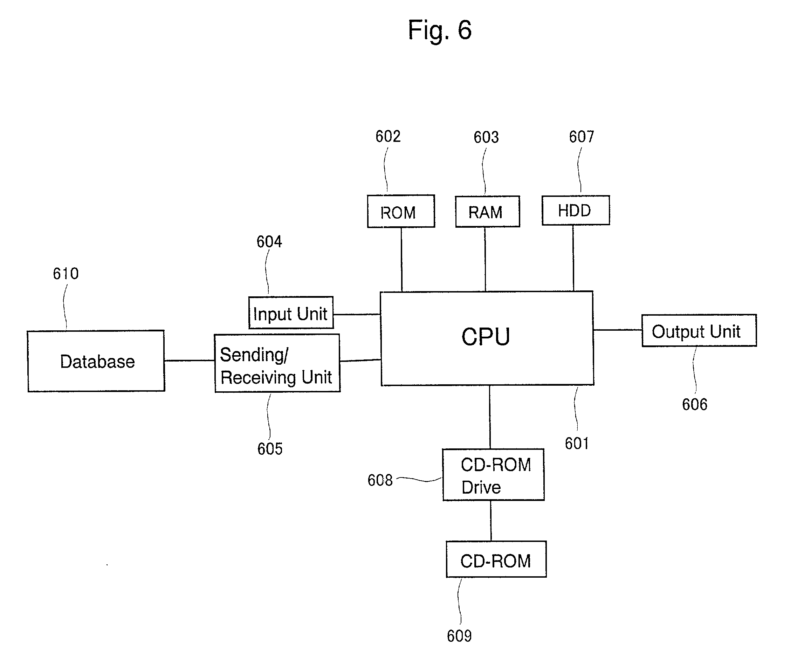

One embodiment of the identification system of the invention is illustrated in a block diagram (Fig. 6).

The identification system shown in Fig. 6 is equipped with CPU 601, ROM 602, RAM 603, Input Unit 604, Sending/Receiving Unit 605, Output Unit 606, Hard Disk Drive (HDD) 607 and CD-ROM Drive 608.

CPU 601 controls the cell, etc. identification system entirely and executes the identification processing described below according to the programs stored in ROM 602, RAM 603 or HDD 607. ROM 602 stores programs, etc. that instruct processing necessary for the operation of the cell, etc. identification system. RAM 603 temporarily stores those data necessary for executing the identification processing. Input Unit 604 is composed of a keyboard, mouse, etc. and operated, e.g., for inputting necessary conditions for the execution of the identification processing. Sending/Receiving Unit 605 sends data to or receives data from External Database 610, etc. through communication circuits, based on instructions from CPU 601. Output Unit 606 displays various conditions that were input from the Input Unit 604, and information about positions or coordinates of spots or bands, intensities of spots or bands, etc., based on instructions from CPU 601. As the Output Unit 606, a computer display unit or a printer may be enumerated. HDD 607 stores cell or tissue identification programs or information on methylation patterns such as bands or spots and, based on instructions from CPU 601, reads out the stored programs or data and stores them, e.g., in RAM 603. Based on instructions from CPU 601, CD-ROM Drive 608 reads out programs or data from the cell, etc. identification programs stored in CD-ROM 609 and stores them, e.g., in RAM 603.

CPU 601 executes identification of cells, etc. based on the data received from Database 610, while supplying data received from the Input Unit, etc. to Output Unit 606. The Database contains accumulated information about spots that were obtained as described above and digitalized.

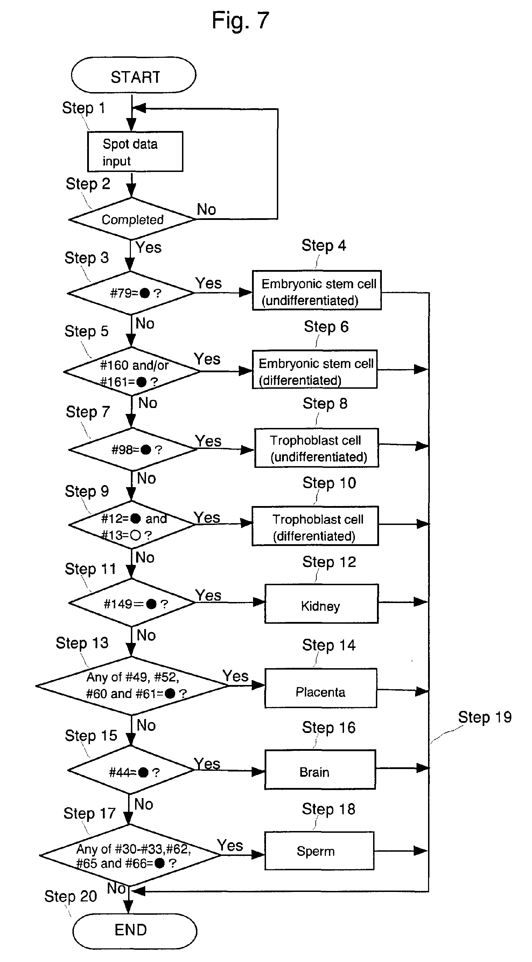

Fig. 7 is a flow chart showing an example of cell identification processing according to the identification program of the invention, wherein information on methylation patterns was analyzed by the RLGS technique. As described in Example 1, a spot pattern as shown in Fig. 3 was obtained for one tissue. Of those positions of spots, 167 positions marked with "○" were selected (Fig. 3). The presence or absence of a spot at these positions was examined on individual cells or tissues, and the results were expressed schematically as shown in Fig. 5. Hereinbelow, one example of identification processing of cells, etc. will be described with reference to the data shown in Fig. 5.

First, spot pattern data obtained by the RLGS technique are input (Step 1). Until data input is completed, Step 1 and Step 2 are repeated. By the data input, information obtained from individual tissues or cells (i.e., the data shown in Fig. 5) is stored in the database. The data about intensities of spots may be expressed with marks such as "●", "ⓞ", "○" or "-" as used in Fig. 5 or with numerical values such as 0, 1, 3, 5, etc.

After completion of the data input, whether or not a strong spot appeared at position 79 is judged (Step 3). If the spot is judged strong (Yes), the tissue or cell tested is identified as embryonic stem cell (undifferentiated) (Step 4). If it is judged that no spot appeared at position 79 (No), then, whether or not a strong spot appeared at position 160 and/or position 161 is judged (Step 5). If the spot(s) is(are) judged strong (Yes), the tissue or cell tested is identified as embryonic stem cell (differentiated) (Step 6). If it is judged that no spot appeared at both positions 160 and 161 (No), then, whether or not a strong spot appeared at position 98 is judged (Step 7). If the spot is judged strong (Yes), the tissue or cell tested is identified as trophoblast cell (undifferentiated) (Step 8). If it is judged that no spot appeared at position 98 (No), then, whether or not a strong spot appeared at position 12 and no spot appeared at position 13 is judged (Step 9). If it is judged that a strong spot appeared at position 12 and no spot appeared at position 13, the tissue or cell tested is identified as trophoblast cell (differentiated) (Step 10). If the tissue of cell tested exhibited a spot pattern other than those described above (i.e., no spot at position 12, and no spot or strong spot at position 13), then, whether or not a strong spot appeared at position 149 is judged (Step 11). If the spot is judged strong (Yes), the tissue or cell tested is identified as kidney (Step 12). If it is judged that no spot appeared at position 149 (No), then, whether or not a strong spot appeared at any of positions 49, 52, 60 and 61 is judged (Step 13). If it is judged that a strong spot appeared at any of positions 49, 52, 60 and 61, the tissue or cell tested is identified as placenta (Step 14). If it is judged that no spot appeared at any of positions 49, 52, 60 and 61, then, whether or not a strong spot appeared at position 44 is judged (Step 15). If it is judged that a strong spot appeared at position 44, the tissue or cell tested is identified as brain (Step 16). If it is judged that no spot appeared at position 44, then, whether or not a strong spot appeared at any of positions 30, 31, 32, 33, 62, 65 and 66 is judged (Step 17). If it is judged that a strong spot appeared at any of positions 30, 31, 32, 33, 62, 65 and 66, the tissue or cell tested is identified as sperm (Step 18). If it is judged that no spot appeared at any of positions 30, 31, 32, 33, 62, 65 and 66, then, identification processing is terminated (Step 20). The identification processing is also terminated when the identification of a cell or tissue of interest is completed (Step 19).

For cells or tissues other than those exemplified above, a schematic drawing of methylation patterns as shown in Fig. 5 may be prepared. Subsequently, identification processing may be performed according to a program such as shown in the flow chart in Fig. 7.

In the method of the invention for identifying cells, tissues or nuclei, it is important to relate in advance DNA methylation patterns of already identified cells, tissues or nuclei to the DNA methylation pattern of a cell, etc. to be identified. In other words, it is important to select information on the cell, tissue of nucleus to be identified, based on information on DNA methylation patterns of already identified cells, tissues or nuclei. It is preferable to use a computer-readable record medium in which are recorded information on DNA methylation patterns of already identified cells, etc. and information on the DNA methylation pattern obtained on the cell, etc. to be identified. Such a record medium may also contain means for comparing methylation patterns and a program that permits a computer to identify the cell, etc. using the comparison results as an indicator. Specific examples of record media include CD-ROM, hard disks, ROM and RAM.

### PREFERRED EMBODIMENTS OF THE INENTION

Hereinbelow, the present invention will be described in more detail with reference to the following Examples. However, the technical scope of the invention is not limited to these Examples.

### EXAMPLE 1

Analysis of Methylation Patterns by the RLGS Technique In this Example, methylation patterns were analyzed using the RLGS technique as one example.

### (1) Preparation of Genomic DNA

Genomic DNA was prepared as described below according to known methods.

Each of frozen tissue (placenta, kidney and brain) and cell (embryonic stem cell, trophoblast stem cell and sperm) samples derived from C57BL/6 mice (0.5-1 g) was suspended in 25 ml of lysis buffer (150 mM EDTA, 10 mM Tris-HCl, pH 8.0, 1 % SDS) containing 10 mg/ml proteinase K (Merk). The mixture was incubated at 55°C for 20 min. Genomic DNA was extracted twice with equal volume of phenol/chloroform/isoamyl alcohol (50:49:1) and precipitated in ethanol. Then, the precipitate was dissolved in 200 µl of TE solution (10 mM Tris-HCl, 1 mM EDTA, pH 7.6).

### (2) RLGS Technique

Methylation of the carbon at 5' position of cytosine is the only chemical modification found in the genomic DNA of mammals. The state of DNA methylation was analyzed on the above described several cells and tissues derived from mice.

Restriction landmark genomic scanning was carried out based on the known method (Okazaki et al., Proc. Natl. Acad. Sci. USA 92:5610-5614, 1995). The genomic DNA (3.5 µg) in 7 µl of TE solution was treated with 10 units of Klenow fragment (TOYOBO) in the presence of 0.4 µM dGTPs, 0.2 µM dCTP (Amersham), 0.4 µM ddATP and 0.4 µM ddTTP. The resultant DNA was first digested with 20 units of *Not*I or *Bss*HII as a landmark restriction enzyme. Then, the resultant 5' overhanging end was radioactively labeled with 1.3 units of Sequenase Ver. 2.0 (USB Co., Ltd.) in the presence of 0.33 µM [α-³²P] dCTP and 0.33 µM [α-³²P] dGTP (Amersham). The labeled DNA (1.5 µg) was digested with 20 units of *Pvu*II (TaKaRa) and subjected to the first-dimensional electrophoresis (0.9% agarose disc gel, about 23 hr, 230 V). Subsequently, the DNA fragments in the gel were digested with 1000 units of *Pst*I (TaKaRa). Then, the second-dimensional electrophoresis was performed at 150 V for 20 hr. After completion of this electrophoresis, the gel was dried and exposed to X ray film (Kodak XAR5) at -80°C for 7-10 days.

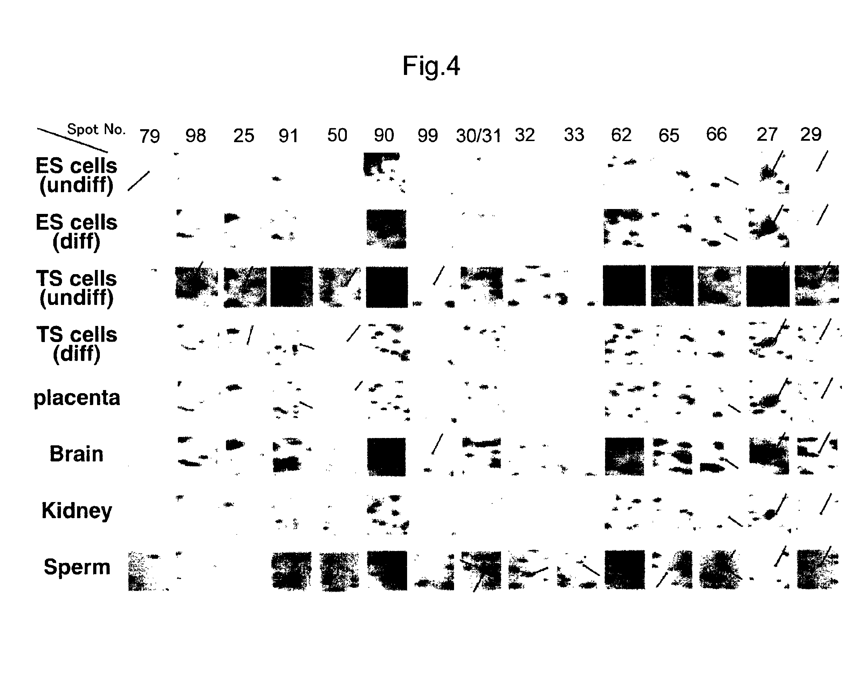

As a result, about 1,000 RLGS spots were detected (Fig. 3). Of these spots, about 85% were spots constantly detected regardless of types of cells or tissues. Those positions at which the pattern of detected spot differs depending on types of cells or tissues were given identification numbers (Nos. 1-167 in Fig. 3). Examples of some spot patterns detected at those positions are shown in Fig. 4. In Fig. 4, spot #79 is specific to embryonic stem cell and not found in other cells or tissues. Spot #98 is specific to trophoblast stem cell. Spot #91 is specific to placenta or trophoblast cell lineage. Spot #99 is observed in brain and differentiated trophoblast cell. Spot #30 is specific to sperm. On the other hand, spot #27 is not observed in sperm but observed in other cells and tissues. Thus, by giving numbers to those RLGS spots at which difference was observed in methylation patterns, difference was found in 167 spots.

A schematic drawing of cell/tissue-specific methylation patterns on the above-mentioned 167 spots is given in Fig. 5. This example of analysis demonstrates that there exist tissue- or cell-specifically methylated or unmethylated regions (at least 167 regions). This means that it is possible to specify the type of a cell or tissue by analyzing the methylation pattern thereof, even if the cell or tissue is an unknown cell or tissue.

### EXAMPLE 2

### Specification of Gene Regions

Genomic DNA was extracted from rat placenta, brain and kidney in basically the same manner as described in section (1), Example 1. Difference in methylation state in gene regions was detected. As a result, difference in methylation pattern was found in 24 genes out of 1033 genes.

Those genes in which difference in methylation pattern had been found were isolated. Their nucleotide sequences were searched through known databases. As a result, citrate transporter and estrogen sulfotransferase were identified as placenta-specific demethylated genes, and sphingolipid kinase and Frizzled as brain-specific demethylated genes.

All the publications, patents and patent applications cited in the present specification are incorporated herein by reference in their entireties.

### EFFECT OF THE INVENTION

According to the present invention, a method of identifying cells, tissues or nuclei using DNA methylation patterns is provided. According to the method of the invention, the type of a cell can be specified even if it is an unknown cell whose characters have not been elucidated sufficiently. Thus, the method of the invention is applicable to the development and establishment of useful cell types.

## Claims

1. A method of identifying a cell, tissue or nucleus, comprising collecting information on the methylation pattern of DNA isolated from said cell, tissue or nucleus and analyzing the resultant information.

2. A method of using information on the methylation pattern of DNA isolated from a test cell, tissue or nucleus as an indicator for producing a cell, tissue or nucleus of interest.

3. A method of specifying gene regions indispensable for producing a cell, tissue or nucleus of interest, using information on the methylation pattern of DNA isolated from a test cell, tissue or nucleus as an indicator.

4. A computer-readable record medium in which a program that permits a computer to function as an identification system for cells, tissues or nuclei has been recorded, said identification system comprising:
(a) means for analyzing information on the methylation pattern of DNA isolated from a test cell, tissue or nucleus; and
(b) means for identifying the cell, tissue or nucleus using the analysis results as an indicator.
